# EUROPÄISCHE PATENTANMELDUNG

(11) **EP 2 359 875 A2**
(43) Veröffentlichungstag der Anmeldung: **24.08.2011**
(21) Anmeldenummer: 11155867.2
(22) Anmeldetag: 15.09.2006
(51) Int. Cl.: A61L 27/14, A61L 27/28, A61L 27/50, A61L 27/54, A61L 31/08

(54) **Bioverträgliches antimikrobielles Filamentmaterial**

(30) Priorität: 15.09.2005 WO PCT/EP2005/009917
(62) Teilanmeldung aus: 06792085.0
(71) Anmelder: Aesculap AG, 78532 Tuttlingen/Donau (DE)
(72) Erfinder: Eggerstedt, Sven, 22527 Hamburg (DE); Odermatt, Erich K., 8200 Schaffhausen (CH); Bargon, Rainer, 78532 Tuttlingen (DE)
(74) Vertreter: Patentanwälte Ruff, Wilhelm, Beier, Dauster & Partner

(57) **Zusammenfassung**

Die Erfindung betrifft ein bioverträgliches Filamentmaterial mit einer antimikrobiellen Ausrüstung, insbesondere in Form einer oberflächlichen Schicht, wobei die Ausrüstung als unspezifisch antimikrobiell wirksame Komponente Polyhexamethylenbiguanid (PHMB) aufweist, wobei das Filamentmaterial als Netz vorliegt, sowie ein Herstellungsverfahren für das Filamentmaterial.

## Beschreibung

Die vorliegende Erfindung betrifft ein bioverträgliches Filamentmaterial mit einer antimikrobiellen Ausrüstung sowie ein Verfahren zur Herstellung des Filamentmaterials und die Bereitstellung des Filamentmaterials für eine Anwendung beim menschlichen oder tierischen Organismus.

Derzeit werden täglich unzählige Eingriffe zu diagnostischen oder therapeutischen Zwecken am menschlichen oder tierischen Körper durchgeführt. Bei diesen Eingriffen werden vielfältigste Implantate, insbesondere Filamentmaterialien, temporär oder dauerhaft in den Körper eingebracht, Untersuchungen mittels mehr oder weniger lange im Körper verweilenden Sonden durchgeführt, die Ver- oder Entsorgung von Stoffen mittels Kathetern gewährleistet sowie vielfältigste Wunden nach medizinischen Eingriffen oder Verletzungen verschlossen. Bei jedem dieser Eingriffe besteht ein erhebliches Risiko der Wundinfektion durch Einschleppung pathogener Keime in den Körper. Insbesondere bei resorbierbaren Filamentmaterialien, beispielsweise für den Wundverschluß, bei denen kein weiterer Eingriff zur Entfernung des resorbierbaren Fremdkörpers beabsichtigt ist, ist es außerordentlich wichtig, dass die Wunde bzw. das Material sowie die Umgebung des Materials bis zur vollständigen Resorption des Materials bzw. zur Heilung der Wunde frei von Infektionen bleibt. Vor allem innerhalb der ersten zwei Wochen nach einem Eingriff besteht ein erhöhtes Risiko von Infektionen durch unerwünschte eingeschleppte Erreger und Vermehrung von pathogenen Keimen aufgrund der geschwächten Immunabwehr und des Wundheilungsprozesses.

Daher wurden bereits viele Anstrengungen unternommen, den Komplikationen durch pathogene Keime vorzubeugen, indem Implantate mit Wirkstoffen ausgerüstet wurden, die die natürliche Immunabwehr unterstützen und die eingeschleppten pathogenen Keime abtöten oder zumindest am Wachstum hindern sollten. So ist beispielsweise aus der US 4,024,872 (US 3,991,766, US 6,528,107) sowie der WO 03/009879 die Imprägnierung bzw. die Zuordnung eines Reservoirs mit einem Breitbandantibiotikum bekannt. Weiterhin beschreiben unter anderem die EP 350147, die US 5,091,442, die WO 03/009879, die US 2002/0193879 sowie die WO 96/22114 die Verwendung von Triclosan in Form von Beschichtungen oder Zusatzstoffen bei der Herstellung von polymeren medizintechnischen Materialien. Die darin beschriebenen medizintechnischen Produkte setzen alle entweder spezifische Antibiotika bzw. Breitbandantibiotika oder den verbreiteten antimikrobiellen Wirkstoff Triclosan zur Bekämpfung von pathogenen Keimen ein. Der Nachteil dieser Wirkstoffe besteht in der allgemein bekannten und mittlerweile in Krankenhäusern sehr gefürchteten Bildung von Resistenzen der pathogenen Mikroorganismen gegen die eingesetzten Antibiotika.

Auch für Triclosan, für welches bisher ein unspezifischer Wirkmechanismus angenommen wurde, ist seit Ende der 90er Jahre ein spezifischer Wirkmechanismus, der in die Fettsäuresynthese der Bakterien eingreift, bekannt geworden. Durch diese Spezifität der Wirkung des Wirkstoffes Triclosan besteht die Gefahr, dass Bakterienstämme bei nicht bakterizid wirksamen Konzentrationen an Triclosan, wie sie vor allem bei der Beschichtung von temporär eingesetzten Produkten wie Kathetern, Handschuhen oder ähnlichem zur Anwendung kommen, Resistenzen entwickeln. Zudem besteht die Gefahr der Ausbildung von Kreuzresistenzen, d.h. dass die Bakterien dadurch auch gegen bestimmte Antibiotika resistent werden können.

Aufgabe der vorliegenden Erfindung ist es daher, ein bioverträgliches Filamentmaterial mit einer unspezifisch aber dennoch hoch wirksamen antimikrobiellen Ausrüstung zur Verfügung zu stellen, die die Besiedelung durch pathogene Keime effektiv verhindert und gleichzeitig die Resistenzbildung gegen die antimikrobielle Ausrüstung verhindert.

Diese Aufgabe wird gelöst durch ein bioverträgliches Filamentmaterial mit einer antimikrobiellen Ausrüstung, insbesondere in Form einer oberflächlichen Schicht, wobei die Ausrüstung als unspezifisch antimikrobiell wirksame Komponente Polyhexamethylenbiguanid (PHMB) aufweist. Das Filamentmaterial einschließlich der antimikrobiellen Ausrüstung liegt vorzugsweise in trockener Form vor.

### Zusammenfassung der Erfindung

Offenbart wird ein bioverträgliches Filamentmaterial mit einer antimikrobiellen Ausrüstung, insbesondere in Form einer oberflächlichen Schicht, wobei die Ausrüstung als unspezifisch antimikrobiell wirksame Komponente Polyhexamethylenbiguanid (PHMB) aufweist.

Bevorzugt liegt es als Geflecht, Gewirk oder Gestrick vor, insbesondere als Gewirk.

Besonders bevorzugt liegt es als Nahtmaterial vor.

Bevorzugt ist, dass die unspezifisch antimikrobiell wirksame Komponente PHMB auf der Oberfläche vorliegt.

Bevorzugt ist das Filamentmaterial während mindestens 7 Tagen, vorzugsweise mindestens während 10 bis 14 Tagen, antimikrobiell wirksam.

Besonders bevorzugt weist das Filamentmaterial ein resorbierbares, insbesondere hydrolysierbares Polymer, auf. Vorzugsweise besteht es aus diesem.

Der Gehalt an PHMB auf der Oberfläche des beschichteten Materials beträgt 0,1 bis 100 µg/cm².

Besonders bevorzugt beträgt der Gehalt an PHMB im antimikrobiell ausgerüsteten Filamentmaterial 2-3000 ppm.

Bevorzugt weist das Material auf der Oberfläche eine von der antimikrobiellen Ausrüstung verschiedene Beschichtung auf.

Bevorzugt beträgt der Anteil an Beschichtungsmaterial am Gesamtmaterial (Filamentmaterial einschließlich Beschichtungsmaterial) 0,1 - 5 Gew.-%.

Bevorzugt handelt es sich bei dem Beschichtungsmaterial um mindestens eines aus der Gruppe umfassend Polyethlyen, Polyester, Silikon und Polyurethan.

Bevorzugt ist das Produkt frei von einer Beschichtung.

Bevorzugt liegt das PHMB in einer in Wasser schwer löslichen Form vor.

Bevorzugt liegt das PHMB in Form von mindestens zwei verschiedenen PHMB-Formen vor, wobei vorzugsweise eine PHMB-Form in Wasser schwer löslich ist.

Bevorzugt liegt die Löslichkeit des schwer löslichen PHMB vorzugsweise bei weniger als 1 g/l Wasser, vorzugsweise weniger als 0,1 g/l Wasser.

Offenbart wird ein Verfahren zur Herstellung eines solchen bioverträglichen Filamentmaterials umfassend die Schritte:
- Herstellung einer Wirkstofflösung aus der unspezifisch antimikrobiell wirksamen Komponente PHMB und einem Lösungsmittel,
- Überführung von PHMB aus der Wirkstofflösung auf und/oder in das Filamentmaterial und
- Trocknung des Filamentmaterials mit dem überführten PHMB.

Bevorzugt wird als Lösungsmittel mindestens ein organisches Lösungsmittel, insbesondere ein Alkohol, Keton und/oder ein aromatisches Lösungsmittel, verwendet, wobei das Lösungsmittel vorzugsweise ein Nichtlöser für das Filamentmaterial ist.

Besonders bevorzugt wird als Lösungsmittel Wasser verwendet.

Bevorzugt beträgt die Konzentration des PHMB in der Wirkstofflösung 0,1 bis 20 Gew.-%, vorzugsweise 0,3 bis 8 Gew.-%.

Bevorzugt wird die Überführung durch Eintauchen des Filamentmaterials in die Wirkstofflösung vorgenommen.

Bevorzugt wird die Überführung durch Aufsprühen der Wirkstofflösung auf das Filamentmaterial vorgenommen.

Bevorzugt wird zusammen mit der antimikrobiell wirksamen Komponente PHMB ein Beschichtungsmaterial gelöst und anschließend auf das Filamentmaterial gebracht.

Bevorzugt beträgt die Konzentration des Beschichtungsmaterials in der Wirkstofflösung 0,5 bis 5 Gew.-%, vorzugsweise 1 bis 3,5 Gew.-%. Bevorzugt wird das Filamentmaterial vor der Auftragung der Wirkstofflösung oberflächenbehandelt, insbesondere durch Sputtern oder durch Plasmaaktivierung, insbesondere durch Plasmaaktivierung.

Offenbart wird die Bereitstellung des bioverträglichen Filamentmaterials wie es beschrieben wurde für eine Anwendung beim menschlichen oder tierischen Organismus, insbesondere beim Wundverschluss und/oder bei Hernien.

Vorteil des mit PHMB versehenen Filamentmaterials ist der unspezifische Wirkungsmechanismus von PHMB gegenüber pathogenen Keimen, welcher die Gefahr der Resistenzbildung gegen die Wirkstoffkomponente PHMB ausschließt. PHMB ist gut verträglich und wird bei der Schwimmbadwasseraufbereitung eingesetzt, ohne dass unerwünschte Nebenwirkungen auf den menschlichen Organismus bekannt sind. Das erfindungsgemäße Filamentmaterial mit einer antimikrobiellen vorzugsweise oberflächlichen Schicht weist als unspezifisch antimikrobiell wirksame Komponente Polyhexamethylenbiguanid (PHMB) auf.

In einer Ausführungsform kann es erfindungsgemäß vorgesehen sein, daß die antimikrobielle Ausrüstung neben PHMB weitere spezifische oder unspezifische antimikrobielle Komponenten aufweist. Vorzugsweise weist das erfindungsgemäße Filamentmaterial als unspezifisch antimikrobiell wirksame Komponente ausschließlich Polyhexamethylenbiguanid auf.

Das Filamentmaterial liegt vorzugsweise in maximal zweidimensionaler Form vor. Es ist vorzugsweise eindimensional oder zweidimensional ausgebildet.

Bei dem Filamentmaterial kann es sich insbesondere um ein mono- oder multifiles Material handeln, wobei ein multifiles Filamentmaterial bevorzugt ist. Erfindungsgemäß kann es sich bei dem Filamentmaterial um ein Geflecht, Gewirk oder Gestrick handeln, wobei ein Gewirk bevorzugt ist.

In einer weiteren Ausführungsform liegt das Filamentmaterial als textiles Flächengebilde, insbesondere als Band oder Netz, beispielsweise als Herniennetz, vor, wobei ein Band besonders bevorzugt ist. Besonders bevorzugt handelt es sich bei dem Filamentmaterial um ein Nahtmaterial.

In einer weiteren Ausführungsform der Erfindung ist die unspezifisch antimikrobiell wirksame Komponente PHMB auf der Oberfläche des Filamentmaterials vorhanden. Vorteilhafterweise besitzt das Filamentmaterial eine antimikrobielle Imprägnierung.

In einer weiteren bevorzugten Ausführungsform ist die unspezifisch antimikrobiell wirksame Komponente im Filamentmaterial und vorzugsweise in der äußeren Schicht der Oberfläche vorhanden. Die unspezifisch antimikrobiell wirksame Komponente PHMB ist vorteilhafterweise durch Lösungsmittel in Schichten unter der Oberfläche von vorzugsweise quellbaren Filamentmaterialien einbringbar.

Mit Vorteil ist das PHMB frei von kovalenten Bindungen zum Filamentmaterial. In einer besonderen Ausführungsform kann PHMB ionische Bindungen zu anionischen, d.h. negativ geladenen Materialien ausbilden.

Gemäß einer weiteren Ausbildung der Erfindung ist das PHMB frei verfügbar und ist insbesondere in das umliegende Körpergewebe diffusionsfähig. Je nach Stärke der Bindung zwischen PHMB und Filamentmaterial kann das PHMB durch Auswaschen aus dem Filamentmaterial freisetzbar sein.

Vorzugsweise ist das Filamentmaterial während mindestens 7 Tagen, vorzugsweise während 10 bis 14 Tage, antimikrobiell wirksam.

Weiterhin kann es erfindungsgemäß erwünscht sein, daß es sich bei dem Filamentmaterial um ein bioresorbierbares Material handelt. Dies kann insbesondere dadurch erreicht werden, daß das Filamentmaterial in-vivo hydrolysierbare Polymere aufweist. Mit Vorteil handelt es sich bei den in vivo hydrolysierbaren Polymeren um Polymere auf der Basis von Lactid, Glykolid, Trimethylencarbonat (TMC) und/oder Dioxanon, wobei ein Filamentmaterial aus Lactid- und/oder Glykolid-Polymer, vorzugsweise aus Glykolid-Polymer, besonders bevorzugt ist. Erfindungsgemäß kann es somit vorgesehen sein, daß der Wirkstoff Polyhexamethylenbiguanid (PHMB) nach vollständiger Resorption bzw. nach Resorption der oberflächlichen Schichten des Filamentmaterials jeweils frei verfügbar und ins Gewebe diffusionsfähig ist. Gemäß dieser besonderen Ausführung ist PHMB in seinem Wirkradius nicht auf die Oberfläche des erfindungsgemäßen Filamentmaterials beschränkt, sondern ist auch in der unmittelbaren Umgebung des Filamentmaterials antimikrobiell wirksam.

Gemäß einer anderen Ausführungsform weist das Filamentmaterial einen nicht bioresorbierbaren Stoff auf, insbesondere ein Polymer, vorzugsweise Polyethylen und/oder Polypropylen. Es ist auch möglich, daß es sich bei dem Filamentmaterial um ein teilweise resorbierbares Material handelt.

Vorteilhafterweise beträgt der Gehalt an PHMB auf der Oberfläche des ausgerüsteten Filamentmaterials zwischen 0,1 und 100 µg/cm². Im Falle von im Filamentmaterial vorhandenem PHMB beträgt der Gehalt an PHMB im antimikrobiell ausgerüsteten Filamentmaterial, insbesondere in einem polymeren Filamentmaterial, vorteilhafterweise 2 -3000 ppm. Gemäß einer weiteren Ausführung weist das Filamentmaterial auf der Oberfläche eine von der antimikrobiellen Ausrüstung verschiedene Beschichtung auf. Die oberflächliche Beschichtung kann resorbierbar oder nicht resorbierbar sein, wobei eine resorbierbare Beschichtung bevorzugt ist. Mit Vorteil weist die resorbierbare Beschichtung ein bioresorbierbares Polymer, insbesondere ein unter in vivo Bedingungen hydrolysierbares Polymer, auf. Bezüglich weiterer Einzelheiten wird auf die obige Beschreibung verwiesen.

Vorteilhaft kann eine Beschichtung zur Erhöhung der Gleitfähigkeit des Filamentmaterials, insbesondere eines Nahtmaterials, vorgesehen sein, um die Handhabung zu erleichtern. Es ist möglich, daß ein nicht resorbierbares Filamentmaterial eine Beschichtung aus einem resorbierbaren Material aufweist. Durch das resorbierbare Beschichtungsmaterial ist die Abgabe und Diffusion der unspezifisch antimikrobiell wirksamen Komponente PHMB von der Oberfläche des Filamentmaterials in das umliegende Gewebe steuerbar, indem das PHMB erst nach der Resorption des Beschichtungsmaterials wirksam ist.

In einer anderen Ausführungsform handelt es sich bei dem Beschichtungsmaterial um ein nicht resorbierbares Material. Dabei ist die Beschichtung aus dem nicht resorbierbaren Material vorteilhafterweise über die gesamte Fläche der Beschichtung mit Durchtrittsöffnungen zur verzögerten und kontrollierten Abgabe der unspezifisch antimikrobiell wirksamen Komponente PHMB in das Gewebe versehen, um den Kontakt zwischen der unspezifisch antimikrobiell wirksamen Komponente auf oder in dem Filamentmaterial und dem umliegenden Gewebe zu gewährleisten.

Mit Vorteil beträgt der Anteil an Beschichtungsmaterial am Gesamtmaterial (Filamentmaterial einschließlich Beschichtungsmaterial) 0,1 bis 5 Gew.-%, entsprechend 1.000 ppm bis 50.000 ppm.

Bei dem Beschichtungsmaterial handelt es sich vorzugsweise um mindestens ein Material aus der Gruppe umfassend Polyethylen, Polyester, Silikon und Polyurethan. Besonders bevorzugt besteht das Beschichtungsmaterial aus resorbierbaren Materialien enthaltend Lactid, Glykolid, Trimethylencarbonat (TMC), ε-Caprolacton (ECL) oder Dioxanon, die als Monomer oder Polymer vorliegen können.

Gemäß einer anderen Ausgestaltung der Erfindung ist das Filamentmaterial frei von Umhüllungen und die unspezifisch antimikrobiell wirksame Komponente PHMB liegt unmittelbar an der Produktoberfläche vor.

Vorzugsweise sind ca. 60 % des PHMB nach dem Einsatz des Filamentmaterials unmittelbar freisetzbar, wohingegen ca. 40 % des PHMB auch nach 14 Tagen noch im Material gebunden sind und somit das Filamentmaterial direkt an seiner Oberfläche weiterhin eine antimikrobielle Aktivität besitzt.

Bei einer bevorzugten Ausführungsform der Erfindung liegt das PHMB mindestens zum Teil in einer in Wasser bzw. in einer in einem wässrigen physiologischen Milieu schwerlöslichen Form vor. Durch Einsatz von schwerlöslichem PHMB kann einerseits die Freisetzungsdauer von PHMB aus dem Filamentmaterial bzw. aus dessen Oberfläche verlängert werden, so dass die antimikrobielle Wirkung auch über einen Zeitraum von mehr als 14 Tagen anhält. Gleichzeitig kann durch eine retadierte Abgabe von PHMB dessen zytotoxische Wirkung gering gehalten werden. Besonders günstig sind Kombinationen von verschiedenen Formen von PHMB. Es ist bekannt, dass PHMB mit verschiedenen Molekulargewichten vorliegen kann. Ferner ist bekannt, dass PHMB in Form von in Wasser gut und weniger löslichen bzw. schwer löslichen Derivaten vorliegen kann. Mischungen von mindestens zwei PHMB Formen sind besonders günstig. Dadurch können die antiseptische Wirkung und deren Dauer eingestellt werden. Bevorzugt ist eine Kombination von mindestens einer PHMB-Variante in schwer löslicher Form und mindestens einer PHMB-Variante in relativ gut löslicher Form. So kann beispielsweise die gut lösliche Form in das Filamentmaterial bzw. die Einzelfasern eindiffundiert sein, wogegen die schwer lösliche Form an der Oberfläche abgelagert ist. Durch Wahl geeigneter Lösungsmittel, insbesondere organischer Lösungsmittel können geeignete Messungen bzw. Lösungsgemische hergestellt werden, die insbesondere im gewünschten Konzentrationsbereich liegen. Geeignete schwerlösliche Formen von PHMB sind Fettsäuresalze mit einem geradzahligen unverzweigten Fettsäurerest von 8 bis 20 Kohlenstoffatomen. Die Löslichkeit der schwerlöslichen PHMB-Verbindungen liegt vorzugsweise bei weniger als 1 g/l Wasser, vorzugsweise weniger als 0,1 g/l Wasser.

Besteht das Filamentmaterial aus synthetischen organischen Polymeren, was bevorzugt ist, dann wird mit Vorteil das jeweilige Lösungsmittel so ausgewählt dass das Filamentmaterial nicht aufgelöst wird. Eine Quellung kann jedoch in vielen Fällen vorteilhaft sein.

Gegenstand der Erfindung ist weiterhin ein Verfahren zur Herstellung eines Filamentmaterials gemäß der vorliegenden Erfindung, umfassend die Schritte:
- Herstellung einer Wirkstofflösung aus der unspezifisch antimikrobiell wirksamen Komponente PHMB und einem Lösungsmittel,
- Überführung von PHMB aus der Wirkstofflösung auf und/oder in das Filamentmaterial und
- Trocknung des Filamentmaterials mit dem überführten PHMB.

Bei dem Lösungsmittel kann es sich insbesondere um mindestens ein organisches Lösungsmittel, beispielsweise einen Alkohol, ein Keton oder ein aromatisches Lösungsmittel, handeln. Besonders bevorzugt sind die Lösungsmittel Isopropanol, Ethylacetat, Toluol und/oder Xylol, wobei Ethylacetat besonders bevorzugt ist. In einer weiteren Ausführungsform des erfindungsgemäßen Verfahrens handelt es sich bei dem Lösungsmittel um Wasser. Es ist ebenso möglich, daß Mischungen verschiedener Lösungsmittel, insbesondere der soeben aufgeführten Lösungsmittel, untereinander oder mit Wasser eingesetzt werden.

Die Konzentration des PHMB in der Wirkstofflösung hängt vom Zweck der unspezifisch antimikrobiell wirksamen Komponente ab. Im Falle einer bakteriziden Wirkung ist eine höhere Konzentration als im Falle einer bakteriostatischen Wirkung des PHMBs bevorzugt. Vorzugsweise beträgt die Konzentration des PHMB in der Wirkstofflösung 0,1 bis 20 Gew.-%, insbesondere 0,3 bis 8 Gew.-%.

In einer besonders bevorzugten Ausführungsform wird die Überführung der unspezifisch antimikrobiell wirksamen Komponente PHMB durch Eintauchen des Filamentmaterials in die Wirkstofflösung vorgenommen. Alternativ dazu kann es vorteilhaft sein, die Überführung durch Aufsprühen der Wirkstofflösung auf das Filamentmaterial vorzunehmen.

Bevorzugt handelt es sich bei dem erfindungsgemäßen Verfahren um ein vollautomatisiertes Verfahren, insbesondere um ein vollautomatisiertes Beschichtungsverfahren. So kann insbesondere ein als Nahtmaterial vorliegendes Filamentmaterial ausgehend von einer Abwickelspule vorzugsweise durch eine Tauchöse in einem Tauchbad zwischen zwei sogenannte Abgautschwalzen transportiert werden, deren Anpressdruck variabel eingestellt werden kann. Das Nahtmaterial kann anschließend über mehrere bewegliche Metallrollen geführt und insbesondere mittels IR-Strahlung und Konvektionstrockner getrocknet werden. Vor der Fadenaufwicklung passiert das Nahtmaterial vorzugsweise eine sogenannte Traversiereinheit. Der Fadentransport ist vorteilhafterweise auf eine konstante Fadenspannung während des gesamten Wickelvorgangs ausgelegt.

Das Applikationsverfahren der Wirkstofflösung ist abhängig von der Geometrie und von der Materialbeschaffenheit, insbesondere der Saugfähigkeit des Filamentmaterials. Bei geometrisch anspruchsvollen Raumformen hat es sich als vorteilhaft erwiesen, das Filamentmaterial aus allen Raumrichtungen mit der Wirkstofflösung gegebenenfalls mehrmals zu besprühen, wohingegen bei saugfähigen Filamentmaterialien vorzugsweise ein Tauchverfahren zum Tränken des Filamentmaterials mit der Wirkstofflösung durchgeführt wird.

In einer besonderen Ausgestaltung des Verfahrens wird ein quellbares Filamentmaterial in die Wirkstofflösung getaucht und verbleibt so lange in der Lösung, bis das Filamentmaterial bis zur gewünschten Eindringtiefe gequollen ist. Dabei dringt das PHMB mit dem Lösungsmittel in das Innere des Filamentmaterials ein. Anschließend wird das Lösungsmittel durch Trocknen vorzugsweise bei Raumtemperatur oder gegebenenfalls bei höherer Temperatur und/oder vermindertem Druck entfernt, wobei das PHMB auch nach dem Entfernen des Lösungsmittels in den vormals gequollenen Schichten des Filamentmaterials verbleibt.

Gemäß einer weiteren Ausführungsform des Verfahrens kann es sich bei der Wirkstofflösung auch um eine Suspension oder Emulsion des PHMB in einem geeigneten Suspensions- oder Emulsionsmittel handeln, mit der das erfindungsgemäße Filamentmaterial nach dem oben beschriebenen Verfahren versehen wird.

In einer besonderen Ausführungsform wird die unspezifisch antimikrobiell wirksame Komponente PHMB zusammen mit dem erfindungsgemäßen Beschichtungsmaterial gelöst und anschließend auf das Filamentmaterial aufgebracht. Damit ist die antimikrobielle Ausrüstung wie die Beschichtung des Filamentmaterials in einem Arbeitsschritt möglich und gleichzeitig wird eine Abgabe der unspezifisch antimikrobiell wirksamen Komponente PHMB an das umliegende Gewebe sofort nach dem Einsatz des Filamentmaterials gewährleistet. Für den Fall, daß die Beschichtung nach der unspezifisch antimikrobiell wirksamen Komponente aufgetragen wird, ist es besonders vorteilhaft, wenn das Beschichtungsmaterial resorbierbar ist, so daß nach Resorption der Beschichtung der antimikrobielle Wirkstoff (PHMB) freigesetzt werden kann.

Je nach Applikationsverfahren der Wirkstofflösung kann die Konzentration an Beschichtungsmaterial in der Lösung variieren. Bei einer einmaligen Applikation der Wirkstofflösung wird vorteilhafterweise eine höhere Wirkstoffkonzentration gewählt als bei einer sehr langen Exposition des Filamentmaterials, insbesondere beim Tauchen in die Wirkstofflösung. Mit Vorteil beträgt die Konzentration des Beschichtungsmaterials in der Wirkstofflösung 0,5 bis 5 Gew.-%, vorzugsweise 1 bis 3,5 Gew.-%.

Um eine gute Haftung der unspezifisch antimikrobiell wirksamen Komponente PHMB auf dem Filamentmaterial bzw. ein gutes Eindringen der Wirkstofflösung in das Filamentmaterials zu gewährleisten, kann das Filamentmaterial vor der Auftragung der antimikrobiellen Lösung oberflächenbehandelt werden. Vorzugsweise wird das Filamentmaterial vor der antimikrobiellen Ausrüstung einer Oberflächenbehandlung, insbesondere einem sogenannten Sputtern oder einer Plasmaaktivierung, unterworfen, wobei eine Plasmabehandlung besonders bevorzugt ist. Bevorzugt wird für die Plasmaaktivierung das APPLD- (Atmosperic Pressure Plasma Liquid Deposition) Verfahren verwendet. Durch die Oberflächenvorbehandlung wird die Aufnahmefähigkeit und/oder Haftung, insbesondere der unspezifisch antimikrobiell wirksamen Komponente PHMB, auf der Oberfläche des Filamentmaterials verbessert.

Weiterhin umfasst die Erfindung die Bereitstellung eines Filamentmaterials gemäß der vorliegenden Erfindung für eine Anwendung beim menschlichen oder tierischen Organismus. Das Filamentmaterial kann sowohl innerlich als auch äußerlich angewandt werden. Bevorzugt ist die Bereitstellung für eine innere Anwendung in Form eines Nahtmaterials, Bandes oder Netzes, insbesondere Herniennetzes, im menschlichen Organismus. Das erfindungsgemäße Filamentmaterial wird vorzugsweise für einen Wundverschluss und/oder bei Hernien verwendet.

Figurenbeschreibung:
- Figur 1:: Funktionsschaltbild einer Fadenbeschichtungsanlage: Von einer Abwickelspule **1** wird der Faden **2** durch eine Tauchöse **3** in einem Tauchbad **4** zwischen zwei beweglichen Abgautschwalzen **5** transportiert, deren Anpreßdruck variabel eingestellt werden kann. Der Faden **2** wird über mehrere bewegliche Metallrollen **6** geführt und mittels IR-Strahler **7** und Konvektionstrockner **8** getrocknet. Vor der Aufwickelspule **10** passiert der Faden eine Traversiereinheit **9.**
- Figur 2:: Rasterelektronische Aufnahme von einem mit PHMB-HCl beschichteten Safil^{®}-Faden
- Figur 3:: Rasterelektronische Aufnahme von einem mit PHMB-Stearat beschichteten Safil^{®}-Faden

Weitere Merkmale der Erfindung ergeben sich durch die nachfolgende Beschreibung von bevorzugten Ausführungsformen in Form von Beispielen in Verbindung mit den Unteransprüchen. Hierbei können die einzelnen Merkmale der Erfindung alleine oder in Kombination miteinander verwirklicht sein. Die beschriebenen Ausführungsformen dienen lediglich zur Erläuterung und zum besseren Verständnis der Erfindung und sind in keiner Weise einschränkend zu verstehen.

### Material und Methoden

Nitroprussid (Di-Natriumpentacyanonitrosyl(II)-Dihydrat), Kaliumhexacyanaoferrat (II), Natriumhydroxid, Schwefelsäure (1 M), Wasserstoffperoxid (Perhydrol, 30%), Stearinsäure und Eisen(II)sulfat wurden von Merck KGaA (Darmstadt), Boratpuffer (pH=11) von Riedel-de-Haen (Seelze) und Polyhexamethylenbiguanid (kurz: PHMB) von Arch Chemicals GmbH (Ratingen) als 20 %ige Lösung bezogen und für die Versuche auf 15 %, 10 %, 5 % und 1 % herunterverdünnt. Als Nahtmaterial wurde Safil beige USP 2/0 von B. Braun eingesetzt.

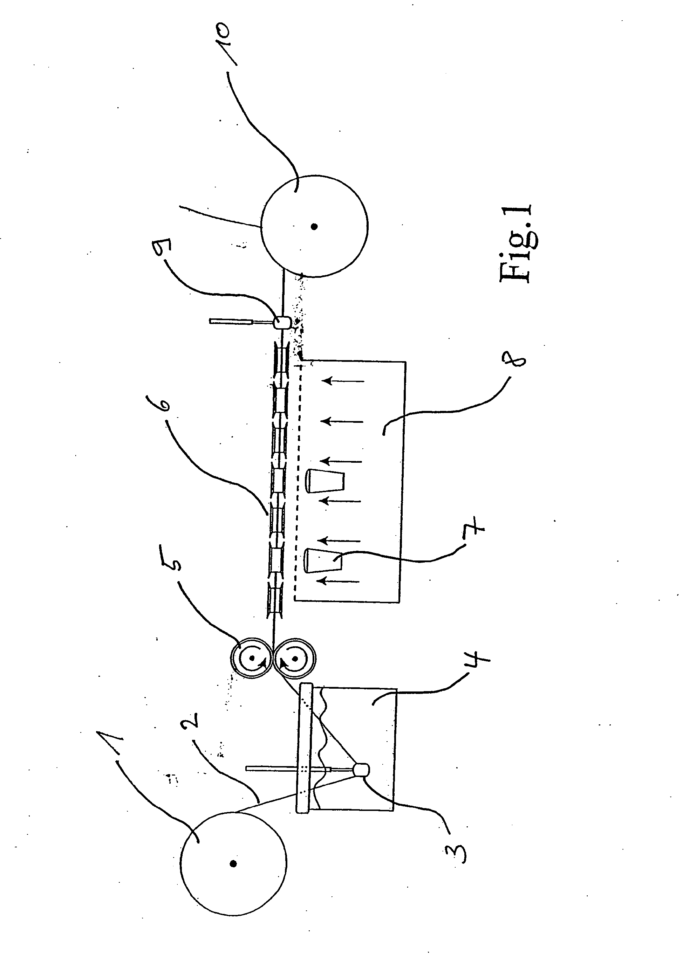

Für die Beschichtungen wurde eine vollautomatische Beschichtungsanlage verwendet (siehe Figur 1.).

Die rasterelektronenmikroskopischen Aufnahmen wurden mit einem Zeiss (Oberkochen) Rasterelektronenmikroskop des Typs 435 VP durchgeführt.

Mikrobielle Analysen wurden bei der Firma medical device testing GmbH (Ochsenhausen) für PHMB-HCl Beschichtungen und bei der Firma Medical Device Service Dr. Rossberger GmbH (Gilching) für die PHMB-stearat Beschichtungen durchgeführt.

Zytotoxizitätstests wurden bei der Firma Medical Device Service Dr. Rossberger GmbH (Gilching) für die PHMB-HCl Beschichtungen und bei NAMSA (Vereinigte Staaten) für die PHMB-stearat Beschichtungen ausgeführt.

Die Beschichtung kann derart erfolgen, dass das Multifilamen ohne Penetration der Beschichtung bedeckt wird, indem eine wässrige oder organische Suspension eines schwerlöslichen PHMB-Salzes eingesetzt wird und/oder dass eine Penetration der Beschichtung ins Innere des Multifilaments stattfindet, wobei die Einzelfilamente mit der Beschichtung ummantelt sind.

### Beispiel 1: PHMB-HCl

### 1.1 Analytik von PHMB-HCl-Lösungen

Die Analytik von PHMB wurde bereits in der Literatur beschrieben (H. Bratt, D. E. Hathway "Characterization of the Urinary Polymer-related Materialform Rats given Poly[biguanidine-1,5-diylhexamethylene hydrochlorid]", Makromol. Chem. 1976, 177, 2591-2606). Die Reagenzlösung wurde wie folgt hergestellt:
10 mL Nitroprussidlösung (10%ig), 10 mL Kaliumhexacyanoferrat(II)-Lösung (10%ig) und 10 mL Natronlauge (10%ig) und 30 mL Wasser wurden gemischt und über Nacht im Kühlschrank gelagert. Die Reagenzlösung kann nicht über mehrere Tage aufbewahrt werden.

### 1.2 Analytik von PHMB-Lösungien

Zur Messung der PHMB-Konzentration in Lösung wurde 1 mL Reagenzlösung mit 5 mL Probe versetzt. Anstelle des Erhitzens auf 45-55 °C, wie in der Literatur beschrieben wird, konnte die Reaktion durch 5 minütiges Beschallen im Ultraschallbad abgeschlossen werden. Die Absorption wurde bei 530 nm mit einem Spektralphotometer bestimmt.

### 1.3 Analytik von PHMB auf Glykolsäurefäden

Zur Analytik des PHMB auf dem Faden muß zuvor die Glykolsäure und der gegebenenfalls im Faden vorhandene Farbstoff D&C violett Nr. 2 als Störkomponenten zerstört werden. Hierzu wurde folgendermaßen verfahren:
6 cm Faden wurden in einen 25-mL-Messkolben gelegt und mit 3 mL Schwefelsäure (1 M) und 3 mL Wasserstoffperoxid (Perhydrol) versetzt. Die Proben werden bei 70°C im Ultraschallbad für 4 h beschallt und dann für einige Stunden im Bad belassen, bis das Bad die Umgebungstemperatur erreicht hat. Mit Natronlauge wird neutralisiert (pH = 7) und zur Zerstörung überschüssiger Glykolsäure 0,1 mL einer Eisen(II)sulfatLösung (0,1 M) zugesetzt und der Kolben für weitere 4 h im Ultraschallbad beschallt. Die Lösung wird auf 25 mL mittels Boratpuffer (pH=11) aufgefüllt. 1 mL obiger Lösung wurde dann mit 0,2 mL Reagenz und mit 1 mL Boratpuffer versetzt und vermessen.

### 1.4 Beschichtung des Fadens mit PHMB-HCl-Lösung

In einer Beschichtungsanlage (vgl. auch Figur 1) wurden etwa 100 m eines Safil^{®} beige Fadens (Polyglykolsäure) der Stärke USP 2/0 kontinuierlich beschichtet. Als Beschichtungsmittel wurde eine 20%ige wässrige PHMB-Lösung eingesetzt. Der Anpressdruck der Abgautschwalzen betrug 100 N, die Beschichtungsgeschwindigkeit 5 m/min, die Trocknungstemperatur 350 °C.

Auf dem Faden verblieben gemäß der analytischen Bestimmung aus 5 Messungen 1786 ± 143 mg/m² PHMB-HCl.

Abhängig von der Beschichtungskonzentration wurden Beschichtungsgehalte auf den Fäden von 90 - 130 mg/m² erzielt.

### 1.5 Oberflächenuntersuchung

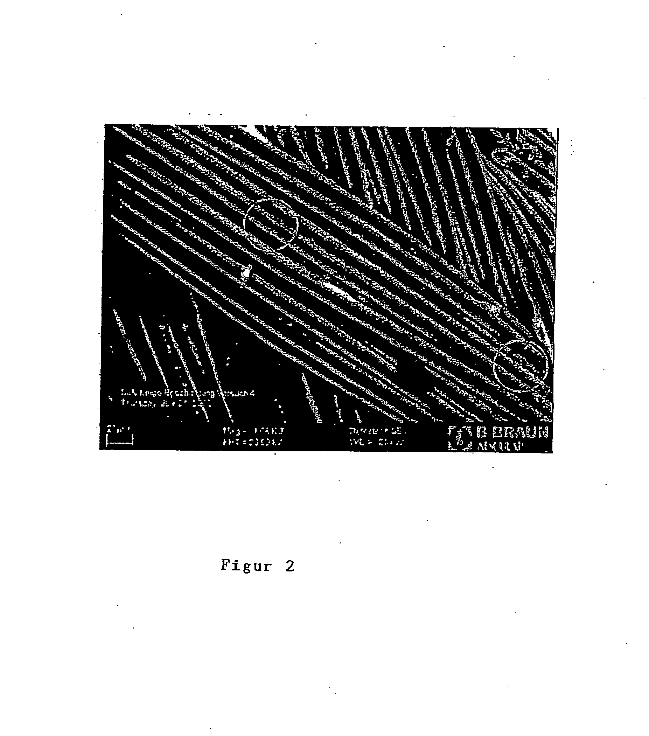

Zur Oberflächenuntersuchung wurden elektronenmikroskopische Aufnahmen durchgeführt. Die Aufnahmen wurden mit einer Beschleunigungsspannung von 20 kV, einem Arbeitsabstand von 25 mm und durch die Detektion von Sekundärelektronen bei 1060 facher Vergrößerung erhalten. Die Kreise deuten Beschichtungsmaterial an (vgl. auch Figur 2).

Die Beschichtung tritt als körniger Belag in Erscheinung (vgl. auch Figur 2). In den Zwischenräumen sind diese deutlich zu erkennen. Die Anlagerung erfolgt relativ gleichmäßig.

### 1.6 Antimikrobielle Wirksamkeit von PHMB-HCl

Eine antimikrobielle Wirksamkeit wurde gemäß einem in der Europäischen Pharmakopöe 2004 beschriebenen Verfahren bestimmt. Hierzu wurden 1,00 Gramm Safil der Fadenstärke USP 2/0, welches mit PHMB-Lösung beschichtet wurde, in 5 ml physiologische Kochsalzlösung überführt. Anschließend wurde die Lösung mit 10⁶ KBE (Kolonien bildende Einheiten) pro g Nahtmaterial beimpft, suspendiert und nach einer Stunde, nach 24 Stunden und nach 72 Stunden Expositionszeit bei Raumtemperatur die Keimzahlen in der Suspension bestimmt. Hierzu wurden pro Zeitpunkt, pro Keim und pro Konzentration 0,5 ml entnommen und je 100 µl und 10µl direkt auf Agarplatte ausplattiert und der Rest membranfiltriert. Die Keimzahlbestimmung erfolgte nach Inkubation bei hohen Keimzahlen (> 200 KBE) auf Agarplatten, bei niedrigen zu erwartenden Keimzahlen durch die Methode der Membranfiltration.

**Tabelle 1. Safil beige USP 2/0 mit 15%iger PHMB-HCl-Lösung beschichtet .**

| **Zeit (Tage)** | P. aeruginosa ATCC 9027 | MRSA ATCC 33592 | C. albicans ATCC 10231 | MRSE CIP 105810 | E. coli ATCC 8739 |
|---|---|---|---|---|---|
| **KMZ (Inokulum)** | 1,20 x 10⁶ | 1,60 x 10⁶ | 4,00 x 10⁵ | 2,60 x 10⁶ | 7,60 x 10⁶ |
| **KMZ nach 1 h** | 2 | 9 | 2 | 15 | 17 |
| **KMZ nach 24 h** | 0 | 0 | 0 | 6 | 0 |
| **KMZ nach 72 h** | 0 | 0 | 0 | 0 | 0 |
| **Log₁₀ Reduktion** | 6,1 | 6,2 | 5,6 | 6,4 | 6,9 |

| | | | | | |
|---|---|---|---|---|---|
| KMZ = Keimzahl | | | | | |

**Tabelle 2. Safil beige USP 2/0 mit 10%iger PHMB-HC1-Lösung beschichtet**

| **Zeit (Tage)** | P. aeruginosa ATCC 9027 | MRSA ATCC 33592 | C. albicans ATCC 10231 | MRSE CIP 105810 | E. coli ATCC 8739 |
|---|---|---|---|---|---|
| **KMZ (Inokulum)** | 1,20 x 10⁶ | 1,60 x 10⁶ | 4,00 x 10⁵ | 2,60 x 10⁶ | 7,60 x 10⁶ |
| **KMZ nach 1 h** | 2 | 9 | 4 | 57 | 64 |
| **KMZ nach 24 h** | 0 | 0 | 1 | 3 | 0 |
| **KMZ nach 72 h** | 0 | 0 | 0 | 0 | 0 |
| **Log₁₀ Reduktion** | 6,1 | 6,2 | 5,6 | 6,4 | 6,9 |

| | | | | | |
|---|---|---|---|---|---|
| KMZ = Keimzahl | | | | | |

**Tabelle 3. Safil beige USP 2/0 mit 5%iger PHMB-HCl-Lösung beschichtet**

| **Zeit (Tage)** | P. aeruginosa ATCC 9027 | MRSA ATCC 33592 | C. albicans ATCC 10231 | MRSE CIP 105810 | E. coli ATCC 8739 |
|---|---|---|---|---|---|
| **KMZ (Inokulum)** | 1,20 x 10⁶ | 1,60 x 10⁶ | 4,00 x 10⁵ | 2,60 x 10⁶ | 7,60 x 10⁶ |
| **KMZ nach 1 h** | 13 | 13 | 11 | 163 | 35 |
| **KMZ nach 24 h** | 0 | 0 | 0 | 2 | 0 |
| **KmZ nach 72h** | 0 | 0 | 0 | 0 | 0 |
| **Log₁₀ Reduktion** | 6,1 | 6,2 | 5,6 | 6,4 | 6,9 |

| | | | | | |
|---|---|---|---|---|---|
| KMZ = Keimzahl | | | | | |

**Tabelle 4. Safil beige USP 2/0 mit 1%iger ,PHUM-HCl-Lösung beschichtet**

| **Zeit(Tage)** | P.aeruginosa ATCC 9027 | MRSA ATCC 33592 | C. albicans ATCC 10231 | MRSE CIP 105810 | E. coli ATCC 8739 |
|---|---|---|---|---|---|
| **KMZ (Inokulum)** | 1,20 x 10⁶ | 1,60 x 10⁶ | 4,00 x 10⁵ | 2,60 x 10⁶ | 7,60 x 10⁶ |
| **KMZ nach 1 h** | 37 | 22 | 40 | 142 | 108 |
| **KMZ nach 24 h** | 0 | 0 | 0 | 5 | 0 |
| **KMZ nach 72 h** | 0 | 0 | 0 | 0 | 0 |
| **Log₁₀ Reduktion** | 6,1 | 6,2 | 5,6 | 6,4 | 6,9 |

| | | | | | |
|---|---|---|---|---|---|
| KMZ = Keimzahl | | | | | |

Eine effiziente antimikrobielle Wirksamkeit gegen gram-positive und gram-negative Bakterien und gegen einen Blastomyceten wurde somit bereits für eine Beschichtungskonzentration von 1,00 g/l PHMB-HCl nachgewiesen.

### 5. Cytotoxizität von PHMB-HCl

Der Test wurde entsprechend der Norm EN ISO/IEC 17025 durchgeführt. Die Fadenstücke wurden lichtgeschützt mit dem DMEM-FBS Zellkulturmedium für 7 Tage bei 37 ± 2°C extrahiert. Das absorbierte DMEM-FBS wurde zu einem Volumen aufgefüllt, so dass das Oberflächen/Volumen-Verhältnis 9 cm²/ml entsprach. Als Negativkontrolle wurde DMEM-FBS ohne Testmaterial für 7 Tage bei 37 ± 2°C inkubiert. 7,5 % v/v DMSO wurde als Positivkontrolle eingesetzt. Extrakte und negative Kontrollen wurden in fünf Schritten mit DMEM-FBS Lösung verdünnt (Verdünnungsverhältnis 2:3). 100 µl der verdünnten Lösungen der Extrakte und der Negativkontrollen sowie 100 µl der Positivkontrollen wurde dreifach in Vertiefungen einer Porzellanplatte hinzugefügt. Dann wurden 50 µl einer frisch zubereiteten Zellsuspension (7;5 x 10⁴ Zellen/ml) zu allen Vertiefungen ausser denen zur Hintergrundsbestimmung hinzugefügt. Die Endkonzentrationen der Extrakte waren somit 66,7 %, 44,5 %, 29,6 %, 19.8 %, 13.2 % und 8,8 % v/v. Die Platten wurden sodann 72 ± 2 Stunden in angefeuchteter Luft (5 % CO2/95% Luft) bei 37 ± 2°C inkubiert). Danach wurde der Proteingehalt der Proben colorimetisch bei einer Wellenlänge von 550 nm bestimmt (BCA Assay Methode).

Als Testorganismen wurden L929 Zellen (DSM ACC2, Mausfibroblasten, Klon des Stangs L) eingesetzt. Das Kulturmedium (Dulbecco's modified Eagle Medium, DMEM) wurde mit 10% fetalem Kälberserum (FBS) überschichtet, 100 U/ml Penicillin (P) und 100 mg/ml Steptomycin (S). DMSO wurde von Merck (Darmstadt) bezogen. FBS und P/S von Biochrom (Berlin) und BCA Protein Quantifikationskit von Interchim (Frankreich).

**Tabelle 5. Safil beige USP 2/0 mit 1%iger PHMB-Lösung beschichtet**

| **Probe / Verdünnung** | **Wachstumshemmung [%]** |
|---|---|
| **Positivkontrolle** | 81 |
| **Negativkonfrolle** | 0 |
| **66,7%** | 10 |
| **44,5%** | 5 |
| **29,6%** | 3 |
| **19,8%** | 3 |
| **13,2%** | 2 |
| **8,8%** | 0 |

Safilfäden, die mit Lösungen höherer Konzentration (5%, 10%, 15%) beschichtet wurden, zeigen eine gewisse Zytotoxizität.

Sowohl die antimikrobiellen Tests als auch die Zytotoxizitätsuntersuchungen zeigen, dass eine Beschichtungskonzentration von kleiner 5% insbesondere von 1 % als Beschichtungslösung besonders geeignet ist.

Beispiel 2: PHMB-Stearat

### 2.1. Synthese von PHMB-Stearat

In einer Standardrührapparatur wurden 900 mL Wasser mit 10,8 g Natriumhydroxid-Plätzchen, 88,8 g Stearinsäure verrührt und mit 300 mL PHMB-HCl-Lösung (20%ig) gemischt. Dann wurde die Suspension 2 h bei 80 °C erhitzt, dann im Eisbad auf 4 °C abgekühlt und filtriert. Dreimaliges Resuspendieren in Wasser mit anschließender Filtration und eine Lyophilisation über Nacht lieferte ein amorphes Pulver, welches gemäß IR-spektroskopischer Reinheitsbestimmung frei von Verunreinigungen war.

### 2.2 Beschichtung des Fadens mit PHMB-Stearat

In einer Beschichtungsanlage (vgl. auch Figur 1) wurden etwa 100 m eines Safil^{®} beige Fadens der Stärke USP 2/0 kontinuierlich beschichtet. Als Beschichtungsmittel wurde eine 20%ige vollständig gesättigte PHMB-Stearatlösung in Toluol eingesetzt. Der Anpressdruck der Abgautschwalzen betrug 100 N, die Beschichtungsgeschwindigkeit 5 m/min, die Trocknungstemperatur 350 °C. Eine Konzentrationsbestimmung konnte mit der in Beispiel 1 beschriebenen Analytik nicht durchgeführt werden.

Eine Schätzung ergibt auf dem Faden eine Konzentration von ca. 90 ± 20 mg/m2_{.}

Die PHMB-stearat ist sehr schlecht in Wasser löslich (<0,1 g/l) jedoch mäßig in Chloroform löslich (ca. maximal 30 g/I).

### 2.3 Oberflächenuntersuchung

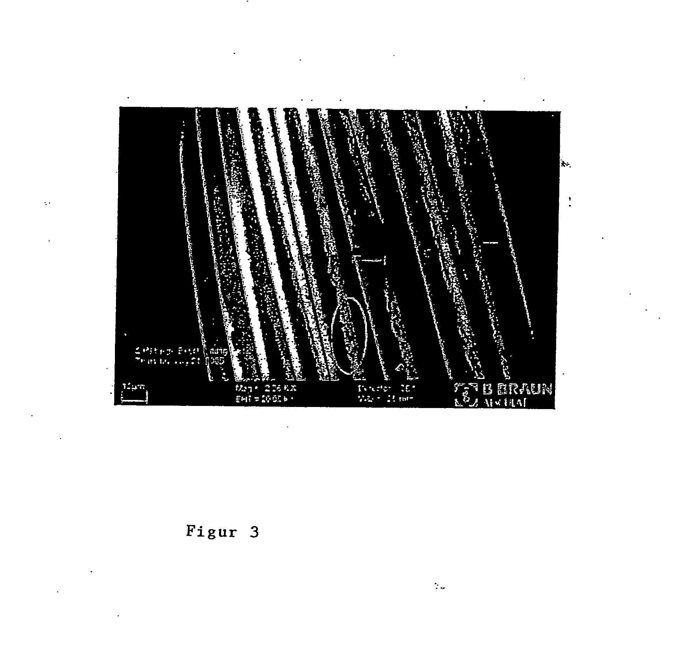

Zur Oberflächenuntersuchung wurden elektronenmikroskopische Aufnahmen durchgeführt. Die Aufnahmen wurden mit einer Beschleunigungsspannung von 20 kV, einem Arbeitsabstand von 25 mm und durch die Detektion von Sekundärelektronen bei 2060 facher Vergrößerung erhalten. Die Kreise in der Abbildung deuten Beschichtungsmaterial an (vgl. auch Figur 3).

Die Beschichtung wirkt einheitlich. Es sind keine Körner und keine Ablagerungen zwischen den Filamenten zu erkennen (vgl. auch Figur 3). Die Anlagerung der Beschichtung ist gleichmäßig.

### 2.4 Antimikrobielle Wirksamkeit

Eine antimikrobielle Wirksamkeit wurde nach 24 stündiger Einlagerung eines 10 cm langen Fadenstücks der oben erwähnten Probe in 20 mL Phosphat gepufferte Saline (pH=7) nach Inokulation mit 5 x 10⁴ KBE (Kolonien bildende Einheiten) pro Keim zum Zeitpunkt t=0 nachgewiesen. Die Ergebnisse sind in der folgenden Tabelle 6 aufgelistet.

**Tabelle 6. Safil beige USP 2/0 mit gesättigter PHMB-Stearatlösung beschichtet.**

| **Zeit (Tage)** | P. aeruginosa ATCC 9027 | S. aureus ATCC 6538 | C. albicans ATCC 10231 | S. epidermidis ATCC 12228 | E. coli ATCC 8739 |
|---|---|---|---|---|---|
| **KMZ (Inokulum)** | 3,50 x 10⁴ | 4,20 x 10⁴ | 3,07 x 10⁴ | 9,75 x 10⁴ | 3,47 x 10⁴ |
| **KMZ nach 0 h** | 7,90 x 10³ | 5,00 X 10⁴ | 3,25 x 10⁴ | 8,10 x 10⁴ | 1,20 x 10⁴ |
| **KMZ nach 24 h** | 1,35 x 102 | 2,65 x 10³ | 2,80 x 10³ | 5,95 x 10³ | 1,70 x 10³ |
| **Log₁₀ Reduktion** | 2,4 | 1,2 | 1,0 | 1,2 | 1,3 |

| | | | | | |
|---|---|---|---|---|---|
| KMZ = Keimzahl | | | | | |

### 2.5 Zytotoxizität von PHMB-Stearat

Zur Bestimmung der Zytotoxizität wurde ein Extrakt des Testartikels hergestellt mit dem Minimum Essential Medium, dem 5% fetales Kälberserum und 2% Antibiotika (1X MEM) hinzugegeben wurde. Dieser Testextrakt wurde auf drei separate konfluente Monolayer von L-929 Mausfibroblasten aufgetragen, die zuvor in angefeuchteter Luft (5% CO2/95% Luft) bei 37 ± 2°C propagiert wurden. Drei separate Monolayer wurden bei 37°C inkubiert in der Anwesenheit von 5% CO2 für 48 Stunden. Die Monolayer in der Testprobe (Safilfäden), in der Positivkontrolle (DMSO) und der Negativkontrolle (Probe ohne Fäden) wurden mikroskopisch nach 48 Stunden untersucht, um Veränderungen in der Zellmorphologie festzustellen. Es konnten in Tests keinerlei Anzeichen für Zytotoxizität festgestellt werden. Auf einer Skala von 0 bis 4 wurde das Testmaterial mit 0 klassifiziert.
0 = keine Zytotoxizität
1 = leichte Zytotoxizität (< 20% Wachstumshemmung)
2 = mittlere Zytotoxizität ( 20 - 50 % Wachstumshemmung
3 = mäßige Zytotoxizität (50 - 70% Wachstumshemmung
4 = schwere Zytotoxizität (70 - 100 % Wachstumshemmung)

Aus Tabelle 6 geht hervor, daß die Beschichtung eine gegenüber bakteriellen Erregern wachstumshemmende Wirkung besitzt.

## Patentansprüche

1. Bioverträgliches Filamentmaterial mit einer antimikrobiellen Ausrüstung, insbesondere in Form einer oberflächlichen Schicht, wobei die Ausrüstung als unspezifisch antimikrobiell wirksame Komponente Polyhexamethylenbiguanid (PHMB) aufweist, **dadurch gekennzeichnet, dass** das Filamentmaterial als textiles Flächengebilde, insbesondere als Netz oder Band, vorliegt.

2. Filamentmaterial nach Anspruch 1, **dadurch gekennzeichnet, daß** es als Geflecht, Gewirk oder Gestrick vorliegt, insbesondere als Gewirk.

3. Filamentmaterial nach Anspruch 1 oder 2, **dadurch gekennzeichnet, daß** die unspezifisch antimikrobiell wirksame Komponente PHMB auf der Oberfläche vorliegt.

4. Filamentmaterial nach einem der vorhergehenden Ansprüche, **dadurch gekennzeichnet, daß** es ein resorbierbares, insbesondere hydrolysierbares Polymer, aufweist vorzugsweise aus diesem besteht, oder einen nicht bioresorbierbaren Stoff aufweist.

5. Filamentmaterial nach einem der vorhergehenden Ansprüche, **dadurch gekennzeichnet, daß** der Gehalt an PHMB auf der Oberfläche des beschichteten Materials 0,1 bis 100 µg/cm² beträgt.

6. Filamentmaterial nach einem der vorhergehenden Ansprüche, **dadurch gekennzeichnet, daß** der Gehalt an PHMB im antimikrobiell ausgerüsteten Filamentmaterial 2-3000 ppm beträgt.

7. Filamentmaterial nach einem der vorhergehenden Ansprüche, **dadurch gekennzeichnet, daß** das Material auf der Oberfläche eine von der antimikrobiellen Ausrüstung verschiedene Beschichtung aufweist.

8. Filamentmaterial nach einem der vorhergehenden Ansprüche, **dadurch gekennzeichnet, dass** das PHMB in einer in Wasser schwer löslichen Form vorliegt.

9. Filamentmaterial nach einem der vorhergehenden Ansprüche, **dadurch gekennzeichnet, dass** das PHMB in Form von mindestens zwei verschiedenen PHMB-Formen vorliegt, wobei vorzugsweise eine PHMB-Form in Wasser schwer löslich ist.

10. Filamentmaterial nach Anspruch 8, **dadurch gekennzeichnet, dass** die Löslichkeit des schwer löslichen PHMB vorzugsweise bei weniger als 1 g/l Wasser, vorzugsweise weniger als 0,1 g/l Wasser liegt.

11. Verfahren zur Herstellung eines bioverträglichen Filamentmaterials nach einem der Ansprüche 1 bis 10, umfassend die Schritte:
- Herstellung einer Wirkstofflösung aus der unspezifisch antimikrobiell wirksamen Komponente PHMB und einem Lösungsmittel,
- Überführung von PHMB aus der Wirkstofflösung auf und/oder in das Filamentmaterial und
- Trocknung des Filamentmaterials mit dem überführten PHMB.

12. Verfahren nach Anspruch 11, **dadurch gekennzeichnet, daß** als Lösungsmittel mindestens ein organisches Lösungsmittel, insbesondere ein Alkohol, Keton und/oder ein aromatisches Lösungsmittel, verwendet wird, wobei das Lösungsmittel vorzugsweise ein Nichtlöser für das Filamentmaterial ist.

13. Verfahren nach einem der Ansprüche 11 oder 12, **dadurch gekennzeichnet, daß** als Lösungsmittel Wasser verwendet wird.

14. Verfahren nach einem der Ansprüche 11 bis 13, **dadurch gekennzeichnet, daß** die Überführung durch Eintauchen des Filamentmaterials in die Wirkstofflösung oder durch Aufsprühen der Wirkstofflösung auf das Filamentmaterial vorgenommen wird.

15. Verfahren nach einem der Ansprüche 11 bis 14, **dadurch gekennzeichnet, daß** das Filamentmaterial vor der Auftragung der Wirkstofflösung oberflächenbehandelt wird, insbesondere durch Sputtern oder durch Plasmaaktivierung, insbesondere durch Plasmaaktivierung.
